# EUROPEAN PATENT APPLICATION

(11) **EP 1 034 798 A2**
(43) Date of publication of application: **13.09.2000**
(21) Application number: 00105066.5
(22) Date of filing: 10.03.2000
(51) Int. Cl.: A61L 9/16

(54) **Damping system of organic particles in an aeriform means**

(30) Priority: 10.03.1999 IT MO990040
(71) Applicant: Centro Analisi Technair S.r.l., 41100 Modena (IT)
(72) Inventor: Bortolani, Giuseppe Nando, 41100 Modena (IT); Bonicelli, Ivo Domenico, 42013 Casalgrande (RE) (IT)
(74) Representative: Luppi, Luigi

(57) **Abstract**

A method for damping undesired smells, includes generating fumes containing smelling particles and directing them outwards through a pre-established path (32, 36, 40; 32, 36, 38, 40) along which there is provided subjecting said fumes to a remarkable heating so as to obtain the combustion of said smelling particles; the apparatus for damping undesired smells includes generating means (11, 14) arranged to generate fumes containing smelling particles, path means (32, 36, 40; 32, 36, 38, 40) arranged to advance said fumes from said generating means (11, 14) toward the outer environment; burning means (36) is disposed along said path means (32, 36, 40; 32, 36, 38, 40) for burning said smelling particles.

## Description

The invention relates to a method and an apparatus wherein measures are provided suitable to prevent organic particles dispersed in an aeriform means from spreading over the environment in which the aeriform means is released.

The invention concerns particularly the damping of undesired smells, in particular the smell of fry, but more in general it can also concern the removal of organic particles from various industrial environments, for examples relevant to painting workings, resin treatments or more else.

The prior art includes filters through which fumes are caused to pass, containing aromatic particles forming on their whole unpleasant smells, the propagation of which in the environment is intended to be prevented. The more effective filters include active coals that retain at their inside said particles; however said filters must be frequently substituted in order to maintain an acceptable degree of functionality.

Alternatively, it has been proposed to condense such fumes by lowering their temperature so as to obtain the precipitation of the particles under liquid form. However, this is economically disadvantageous, first of all because a suitable refrigerating system must be provided in order to condensate the fumes, which implies an increase of installation and operation costs.

Furthermore, the treatment of the condensed liquid is difficult, because the liquid is very dense and tends to occlude the ducts through which it is made to flow towards the collection containers; these last must be then periodically cleaned, or substituted, which affects the maintenance costs.

Burning apparatus are further known, wherein an aeriform means containing the particles to be removed is introduced into a chamber in which a suitable fuel is burned so that the whole mass of the aeriform means and of the dispersed particles to be damped is brought to a very high temperature (about 850 °C) and maintained at that temperature for a pre-established time interval. The temperature level and the time interval are chosen in such a way as to allow the dissolution of the particles.

This implies a remarkable consumption of fuel, requires a certain waste of time and makes necessary to rise also the temperature of that portion of the aeriform means that does not include the particles to be damped and, therefore, would not need to be heated.

Therefore, it is desirable to find an efficient system for the damping of organic particles dispersed into a fluid means.

In a more particular aspect, there is the problem of limiting the propagation of smells, in particular fry smells of the frying machines for the automatic delivery of chips: said machines are usually installed into indoor spaces, such as public rooms, trading centres, offices, frequented by people not assigned to cooking workings, spaces where the fry smell results entirely unacceptable.

In a first aspect of the invention, a method is provided for damping organic particles dispersed into a gaseous fluid, comprising generating a gaseous fluid containing organic particles and sending it outwards through a pre-established path, characterized in that, along said path there is provided causing said fumes to pass near heating elements that attract said particles and dissolving said particles when they come in contact with said heating elements.

In a second aspect of the invention, an apparatus is provided for damping organic particles dispersed into a gaseous fluid, including generating means arranged to generate fumes containing smelling particles, path means arranged to advance said fumes from said generating means toward the outer environment, characterized in that thermic treatment means are arranged along said path means to attract the smelling particles towards heating elements of said thermic treatment means and to neutralize them when the smelling particles come in contact with said heating means.

In this way the amount of particles delivered into the environment is strongly reduced.

By a suitable choice of the temperature of the heating elements and their arrangement, that influences the conditions at which smokes are treated, the content of undesired particles is substantially removed.

In particular, the content of smelling particles can really fall under the threshold of the human perception.

In this way it is possible to install automatic chip frying-vending machines also in spaces where the emission of smells is particularly undesirable.

The invention can be better understood and carried out with reference to the enclosed drawings, in which:

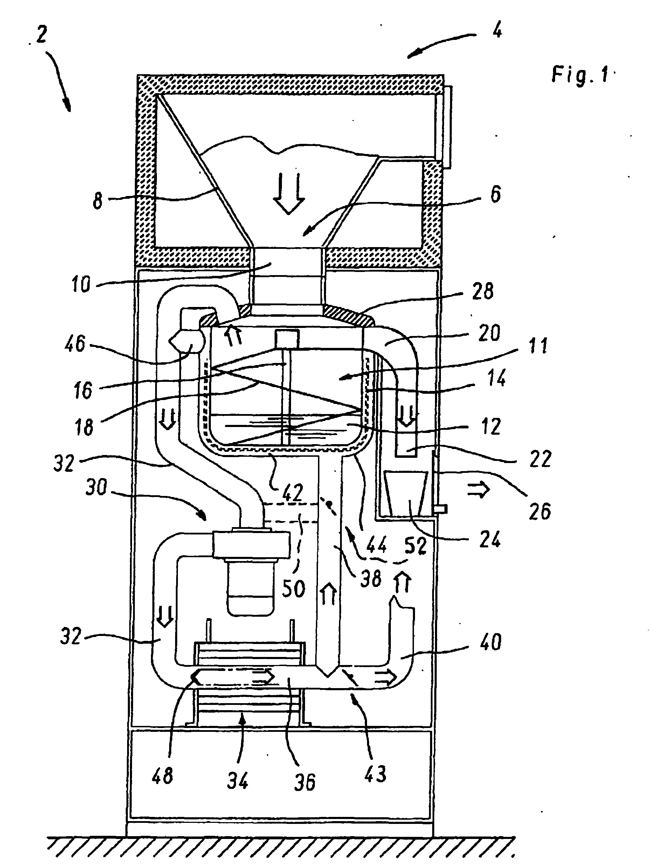

Figure 1 is a schematic vertical section of a chip frying-vending apparatus;
Figure 2 is a broken, schematic and enlarged section of a variant of the treatment path for the fumes.

An apparatus 2, for frying and vending chips, includes a container 4 thermally insulated and thermally coupled to a refrigerating group, not shown, in which the chips to be fried are contained; the chips are advanced towards the outlet 6 of a hopper 8 controlled by closing means 10 that can be opened and closed when tokens or coins are inserted through a slot of a suitable coin box, not shown.

The outlet 6 ends into a container 11, at the bottom of which a frying fluid 12 is contained, electric heating resistors 14 being applied on the outside of the container. Inside the container 11 a screw feeder 18 rotatingly supported to a central vertical axis 16, which is operated, by means not shown, once the chips fallen from the hopper 8 into the container 11 have been fried, so as to extract the chips and to convey them through an outlet 20, controlled by closing means, not shown, from which these last are caused to fall into a cup container 24 through a collection duct 22. The cup container 24 can then be extracted from the machine 2 by opening a door 26.

At the upper side of the container 11 a cover 28 is provided peripherally hermetically sealed along its external edge, the cover 28 being provided with an extraction duct 32 through which fumes are extracted by an extractor fan 30 and sent to a burning group 34. The burning group 34 is constituted by an inner cylindrical treatment chamber 36 communicating upstream with the extraction duct 32, of which constitutes an extension, and downstream with a recovery duct 38.

The extractor fan 30 can be replaced by a compressor by means which it is possible to rise the temperature of the fumes before the treating them into the chamber 36.

The inner treatment chamber 36 has inner walls electrically heated and is crossed by the fumes coming from the extraction duct 32 that are subjected to a temperature rising in said chamber capable of causing a flame-free combustion of the fumes, which causes oxidation of the smelling particles contained therein.

More in particular, the walls 36 can be brought to a temperature of incandescence, so as to attract only the smelling particles and oxidize them when they arrive in contact with the walls themselves.

The smelling particles, once oxidized, leave no solid waste, but only combustion products at a gaseous state, that are expelled outwards without producing remarkable smells.

From the recovery duct 38 a discharge duct 40 is branched through which the already treated fumes are released to the outer environment. Near the intersection between the discharge duct 40 and the recovery duct 38 a flow chocking valve 43 is mounted, which can be operated to vary the ratio between the flow of the treated fumes to be introduced into the recovery duct 38 and the flow of fumes to be released to the environment through the discharge duct 40.

The recovery duct 38 ends into an interspace 42, defined by a covering 44 wrapping the outer side of the container 11 from the bottom and laterally and in which the treated fumes arriving from the recovery duct 38 lick the container 11 in order to deliver heat to the fluid 12 and promote the cooking of the chips contained therein.

The interspace 42 communicates with the extraction duct 32 through a collector 46 that reintroduce the treated fumes into the extraction duct 32 after the fumes have delivered their heat to the container 11.

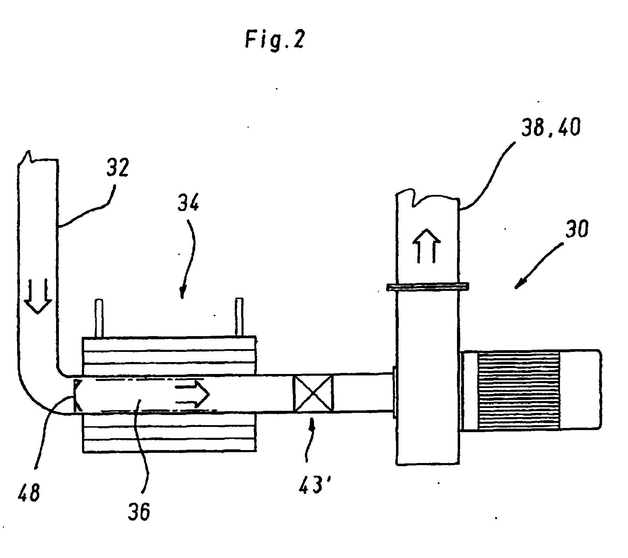

As shown in Figure 2, the extractor fan 30 can be installed downstream the treatment chamber 36 and a valve 43' for chocking the fumes's flow so as to direct the fumes themselves to the discharge duct 40, or to the recovery duct 38.

In this last case, a further discharge duct must be provided, however not shown.

The recovery duct 38 and the collector 46 can be advantageously provided in order to obtain a remarkable energy saving, but they are not essential in order to achieve the desired damping of smells in the fumes.

Furthermore it is possible to insert a by-pass duct 50 between the recovery duct 38 and the extraction duct 32, in order to use a portion of the hot and treated fumes for heating the container 11 while recycling the remaining part thereof for preheating the smokes upstream the fan/compressor 30. With the aid of a further flow chocking valve 52 it is possible to divide the flow of treated fumes to be directed to heat the container 11, or to the extraction duct 32.

Obviously, if it is not necessary to heat the container 11 through the treated fumes, the whole flow of treated fumes can be directed to the extraction duct 32.

The treatment chamber 36 cooperates with a flow diverter 48 so shaped as to divert the fume flow towards the walls of the chamber itself, as shown in the drawing by a dotted line, in order to promote the heat exchange with the walls of the chamber and increase the probabilities that the particles are attracted by the walls of the chamber 36 and oxidized when contacting them.

The flow diverter 48 can have a conical shape, or can be constituted by a fixed blading.

The discharge duct 40 opens to the outside in a region which is not easily accessible by people who is near the machine 2. For example, the discharge duct 40 can be aligned in a vertical direction, so as to cause the treated fumes, still being at a rather high temperature, to be discharged upward.

Furthermore, the duct 40 can be shielded, or insulated with known means, in order to be prevented from causing burns to people who unintentionally touch it.

Obviously, the invention will be carried out not only in the domain of a frying machine for chips, but also in all the civil and industrial environments in which it is necessary to remove unpleasant smells or general organic particles, which may be potentially unhealthy.

## Claims

1. Method for damping organic particles dispersed into a gaseous fluid, including generating a gaseous fluid containing organic particles and directing it outwards through a pre-established path (32, 36, 40; 32, 36, 38, 40), characterized in that, along said path (32, 36, 40; 32, 36, 38, 40) there is provided causing said fumes to pass near heating elements (36) that attract said particles and dissolving said particles when they arrive in contact with said heating elements (36).

2. Method according to claim 1, wherein said dissolving takes place in a chamber (36) through which said gaseous fluid passes.

3. Method according to claim 1, or 2, wherein said path (32, 36, 40) extends between a region near the region where said gaseous fluid is generated and a region where said gaseous fluid is released into the environment, after having been subjected to the action of said heating means (36).

4. Method according to claim 3, wherein said path extends furthermore between a region below the region where said gaseous fluid is subjected to the action of said heating elements (36) and a region (42) near the region where said gaseous fluid is generated.

5. Method according to claim 4, wherein said gaseous fluid, after having been subjected to the action of said heating elements (36), is directed, totally or partially, towards said region where said smokes are generated.

6. Method according to claim 4, or 3, wherein said gaseous fluid, after having been subjected to the action of said heating elements (36), is directed to a region above the region where it is subjected to the action of said heating elements (36).

7. Method according to any of the preceding claims and further including pressurizing said gaseous fluid.

8. Apparatus for damping organic particles in a gaseous fluid, including generating means (11, 14), arranged to generate a gaseous fluid containing organic particles, path means (32, 36, 40; 32, 36, 38, 40; 32, 36, 38, 40, 50) arranged to advance said gaseous fluid from said generating means (11, 14) towards the outer environment, characterized in that, along said path means (32, 36, 40; 32, 36, 38, 40; 32, 36, 38, 40, 50) thermic treatment means (36) is arranged capable of attracting said particles towards heating elements (36) of said thermic treatment means and for neutralizing them when the particles arrive in contact with said heating elements (36).

9. Apparatus according to claim 8, wherein said path means (32, 36, 40; 32, 36, 38, 40; 32, 36, 38, 40, 50) includes extraction duct means (32) arranged to extract said gaseous fluid from said generating means (11, 14).

10. Apparatus according to claim 8, or 9, wherein said thermic treatment means includes heated chamber means (36) wherein said gaseous fluid is heated up to a temperature at which said particles are oxidized.

11. Apparatus according to claim 10, wherein said chamber means (36) is provided with flow diverter means (48) arranged to divert said gaseous fluid towards walls of said chamber means (36).

12. Apparatus according to any of claims 8 to 11, wherein said path means (32, 36, 40; 32, 36, 38, 40; 32, 36, 38, 40, 50) includes discharge duct means (40) arranged to direct said gaseous fluid towards the outer environment from the outlet of said thermic treatment means (36).

13. Apparatus according to any of claims 8 to 12, wherein said path means (32, 36, 40; 32, 36, 38, 40; 32, 36, 38, 40, 50) includes valve means (43; 43'; 43, 52) limiting the flow of said gaseous fluid.

14. Apparatus according to any of claims 8 to 13, wherein said path means (32, 36, 40; 32, 36, 38, 40; 32, 36, 38, 40, 50) includes recycling duct means (38) arranged to recycle said gaseous fluid from the outlet of said thermic treatment means (36) towards said generating means (11, 14).

15. Apparatus according to any of claims 8 to 14, wherein said path means (32, 36, 38, 40, 50) includes by-pass duct means (50) for directing said fluid from the outlet of said thermic treatment means (36) towards a region of said path above said thermic treatment means (36).

16. Apparatus according to claim 15, wherein said recycling duct means (38) opens into an interspace (42) wrapping said generating means (11, 14).

17. Apparatus according to claim 16 wherein said path means (32, 36, 40; 32, 36, 38, 40) further includes collector means (46) that connects said interspace (42) with said extraction duct means (32).

18. Apparatus according to any of claims 8 to 17, wherein pressurizing means (30) is arranged along said path means (32, 36, 40; 32, 36, 38, 40; 32, 36, 38, 40, 50) in order to pressurize said gaseous fluid.

19. Apparatus according to any of claims 8 to 18, wherein said generating means (11, 14) includes a container (11) wherein chips are fried.
